# EUROPEAN PATENT SPECIFICATION

(11) **EP 3 569 201 B1**
(45) Date of publication and mention of the grant of the patent: **05.04.2023**
(21) Application number: 18172851.0
(22) Date of filing: 17.05.2018
(51) Int. Cl.: A61F 2/95, A61F 2/24, A61M 25/01

(54) **FORCE LIMITING HANDLE ASSEMBLY AND HEART CATHETER BASED DELIVERY SYSTEM**
KRAFTBEGRENZUNGSGRIFFANORDNUNG UND HERZKATHETERBASIERTES FÖRDERSYSTEM
ENSEMBLE DE POIGNÉE DE LIMITATION DE FORCE ET SYSTÈME D'ADMINISTRATION PAR CATHÉTER CARDIAQUE

(43) Date of publication of application: 20.11.2019
(73) Proprietor: Creganna Unlimited Company, Ballybrit, Galway H91 VN2T (IE)
(72) Inventor: MURPHY, Brian, Galway, H91 V8XA (IE)
(74) Representative: Grünecker Patent- und Rechtsanwälte PartG mbB

(56) References cited:
- EP-A1- 2 832 318
- WO-A1-2007/139457
- US-A1- 2012 150 106
- US-A1- 2013 345 801

## Description

The present invention relates to a handle assembly for a structural heart catheter based delivery system, a delivery system for delivering an intravascular device to targeted anatomy within the heart such as at the mitral annulus, and further relates to a method of fabricating such a handle assembly. The present invention further relates to a method of testing such a delivery system.

Intravascular medical procedures allow the performance of therapeutic treatments in a variety of locations within a patient's body while requiring only relatively small access incisions. An intravascular procedure may, for example, eliminate the need for open-heart surgery, reducing risks, costs, and time associated with an open-heart procedure. The intravascular procedure also enables faster recovery times with lower associated costs and risks of complication. An example of an intravascular procedure that significantly reduces procedure and recovery time and cost over conventional open surgery is a heart valve replacement or repair procedure in which an artificial valve or valve repair device is guided to the heart through the patient's vasculature. For example, a catheter is inserted into the patient's vasculature and directed to the inferior vena cava. The catheter is then urged through the inferior vena cava toward the heart by applying force longitudinally to the catheter. Upon entering the heart from the inferior vena cava, the catheter enters the right atrium. The distal end of the catheter may be deflected by one or more deflecting mechanisms, which can be achieved by tension cable, or other mechanisms positioned inside the catheter. Precise control of the distal end of the catheter allows for more reliable and faster positioning of a medical device and/or implant and other improvements in the procedures.

An intravascularly delivered device needs to be placed precisely to ensure a correct positioning of the medical device, which is essential for its functionality, as the device may be difficult to reposition after the device is fully deployed from the delivery system. Additionally, the ability to recapture a partially deployed device is desirable in the event that the distal end of the catheter moves relative to the target location and compromises the precise positioning of the device.

It is known to use an actuating wheel element that is manually actuated to perform a longitudinal movement of, for instance, a deployment mechanism. However, the problem may occur that a too high mechanical force is applied to the wheel element, so that the handle assembly and/or the delivery system is damaged. This is fatal for the surgery to be performed.

From EP 2 832 318 A1 a handle assembly for an implant delivery device is known for folding or unfolding at least one medical implant by means of at least one tension thread, wherein the handle assembly comprises a drum for winding the tension thread thereon by rotating the drum; a knob to be rotated by a user of the handle assembly in order to fold or unfold the medical implant, the knob being interconnected with the drum such that the drum is rotated when the knob is rotated; and a force limiter for limiting the maximum force or tension that may be applied or is applicable to the tension thread or to the drum by rotating the knob.

WO 2007/139457 A1 discloses a steerable stylet for a medical implantable lead of the type having an inner lumen, into which the steerable stylet is insertable for navigating a distal end of the medical implantable lead to the desired location for attachment to tissue. The steerable stylet comprises a wire, a tube and an actuator. By means of the steerable stylet, the distal portion of the lead may be bent in desirable degree and, when the medical implantable lead is bent, the distal portion of the medical implantable lead is pivotal by means of the actuator to which the proximal end of the wire is non-rotatably connected. According to this document, the tube is connected to the actuator by means of a torque limitation device which, when exceeding a predetermined torque force, will disconnect the torque connection between the tube and the actuator.

US 2012/0150106 A1 discloses a catheter which includes a handle that limits the amount of torque that can be imparted to the body of the catheter. This is intended to reduce the likelihood of catheter failure, damage to tissue, or damage to medical devices introduced into the vasculature via the catheter. The catheter handle includes a grip portion that the practitioner manipulates in order to impart a torque and a torque transmitting portion operably coupled thereto that transmits the torque to the catheter body. A torque limiting mechanism decouples the torque transmitting portion from the grip portion, the body, and/or any pull wires when the torque imparted to the grip portion exceeds a torque threshold, thereby preventing excessive torques from being transmitted to the catheter body and/or pull wires. A practitioner may be able to adjust the torque threshold and may be able to disable the torque limiting mechanism.

US 2013/345801 A1 discloses a valve sizer for determining an appropriate replacement valve size when performing a heart valve replacement procedure. In one version the valve sizer has a hollow shaft with proximal and distal ends. A movable sizing element couples to the distal end of the shaft and is radially expandable between first, contracted and second, expanded positions. An actuator assembly on a handle includes an actuator coupled to a clutch member via a ballspring-detent clutch. A rod extends through the shaft and maintains a fixed distance between the handle and a distal hub in the sizing element. Movement of the actuator causes axial movement of the shaft, thereby causing radial expansion of sizing petals relative to the hub. The clutch slips when a predetermined reaction force from the surrounding valve annulus is met by the petals.

There is still a need for an improved handle assembly that has a higher degree of safety during use, at the same time being robust and economic to manufacture.

This object is solved by the subject matter of the independent claims. Advantageous embodiments of the present invention are the subject matter of the dependent claims.

The present invention is based on the idea that by adding a torque limiting device to the wheel element of a delivery system, it can be avoided that the components of the delivery system reach the breaking point. Thus, it is ensured that the delivery system is not inadvertently destroyed by exerting to much manual force on the wheel element when preparing for or performing surgery. In particular, the present invention provides a handle assembly for a structural heart catheter based delivery system, the handle assembly comprising an actuation unit that is connectable to a delivery member for performing a movement of the delivery member in a longitudinal direction, and an actuating element for manually actuating the actuation unit, wherein the actuating element comprises a wheel element that is rotatable around a rotation axis, wherein the wheel element is attached to the actuation unit via a force limiting coupling unit for disengaging the wheel element from the actuation unit if a mechanical force applied to the wheel element by a user exceeds a threshold value.

This solution has the advantage that the force which is manually delivered to the wheel element is not fully translated into the delivery system, but limited to a chosen threshold value. The level of permissible force may be determined based on the mechanical stability of the used delivery system. For instance, the force limiting coupling unit may be set to disengage before a force of 80 N is reached, if this force is known to cause failure of the catheter.

According to the present invention, the force limiting coupling unit comprises spring-loaded, ball bearings that are engaged with corresponding bearing recesses as long as the applied mechanical force does not exceed said threshold value. By using such a ball-recess coupling a miniature ball bearing slipper clutch can be created which produces similarly satisfactory results in wet and dry conditions. This is particularly important for medical applications.

Each ball bearing comprises a ball plunger, the ball plunger having a body with a spring-loaded ball retained therein. Such ball plungers are generally known and have the advantage that they are well-established parts that can be handled easily, the springs already mounted inside the ball plunger body. However, it is clear for a person skilled in the art that, according to configurations not covered by the present invention, the balls can be resiliently mounted directly at another suitable part of the force limiting coupling unit.

According to the present invention, the wheel element is rotatable around said longitudinal axis of the handle assembly. This allows the wheel element to be formed by a tubular element that is arranged around the force limiting coupling unit. Thereby, a particularly simple structure can be achieved that can be fabricated economically. Furthermore, an ergonomic design can be achieved that is easily actuated. Of course, however, the idea of the present invention may also be used with wheel elements that have rotation axes which extend across to the longitudinal axis of the handle, thereby providing a dial that is more readily visible for the actuating person and can be rotated in a more ergonomic way.

In order to efficiently realize a well-defined torque limitation, the force limiting coupling unit according to the present invention comprises an inner bearing element that is coupled to the actuation unit and an outer bearing element that is accessible for a user and is engaged with the inner bearing element as long as the mechanical force applied by the user is below said threshold value. In particular, the inner bearing element and the outer bearing element may be arranged concentrically with respect to each other around the rotational axis.

An economic way of assembly can be achieved when the outer bearing element comprises at least two segments that are interconnected with each other along an interface that extends along said longitudinal axis. For instance, two half-shells can be provided that are screwed together to form a cylindrical tube shaped outer bearing body. This outer bearing body may be touchable on its outer surface by the user and is provided with a suitable surface finish that improves the grip.

According to an advantageous embodiment of the present invention, the outer bearing element has a tube shape with a first and a second end face plane and comprises a plurality of balls which are spring-loaded along said longitudinal axis, said balls extending from at least one of the first and second end face planes. Accordingly, the inner bearing element may have a tube shape with at least one collar arranged at a peripheral end of the tube around the circumference of the tube, wherein a plurality of bearing recesses are arranged at the collar which engage with said balls. Such an arrangement has the advantage that it works equally well under wet and dry conditions which is important for an application with heart catheter surgery.

In order to allow a continuous torque limitation and re-engagement of the coupling which enables the user to continue the actuation with reduced force, said balls and said bearing recesses are distributed equidistantly around the circumference of the at least one end face plane and the collar, respectively, so that each of the balls disengages from one recess when the force exceeds the threshold values and engage with the adjacent bearing recess. Generally, the threshold value of the force is increased by increasing the amount of balls and by providing the balls with a higher spring force.

According to an alternative of the present invention, the outer bearing comprises a plurality of ball plungers which are arranged along said longitudinal axis. Such ball plungers are generally known and have the advantage that they are well-established parts that can be handled easily, the springs already being mounted inside the ball plunger body.

In order to create a design that can be adapted by a user to particular force threshold values, the outer bearing is equipped, according to an alternative of the present invention, equipped with a particular maximum amount of cylindrical cavities (though holes or blind holes) for receiving a ball plunger each, and the inner bearing is provided with at least the corresponding number of recesses. As the torque limit is determined by the amount of balls, a user can insert as many ball plungers as needed for a particular force threshold value (up to the maximum number where all holes each contain a ball plunger). For lower values of the torque limit, a certain amount of recesses remains empty in each stable position of the force limiting coupling unit. These recesses may accommodate a ball after the inner and outer bearing have changed their radial position with respect to each other if the applied force has exceeded the threshold value. For instance, the design may be chosen to have cavities for up to 32 ball plungers. Of course, any other number of cavities may also be used.

The present invention further relates to a delivery system for intravascularly delivering replacement components, the delivery system comprising a catheter assembly and a handle assembly according to the invention.. For instance, the delivery system can be used with prosthetic heart valve replacement, and more particularly with devices, systems, and methods for transapical and transcatheter delivery of collapsible prosthetic heart valves. Prosthetic heart valves that are collapsible to a relatively small circumferential size can be delivered into a patient less invasively than valves that are not collapsible. For example, a collapsible valve may be delivered into a patient via a tube-like delivery apparatus such as a catheter, a trocar, a laparoscopic instrument, or the like. This collapsibility can avoid the need for a more invasive procedure such as full open-chest, open-heart surgery.

Collapsible prosthetic heart valves typically take the form of a valve structure mounted on a stent. There are two types of stents on which the valve structures are ordinarily mounted: a self-expanding stent and a balloon-expandable stent. To place such valves into a delivery apparatus and ultimately into a patient, the valve must first be collapsed or crimped to reduce its circumferential size. When a collapsed prosthetic valve has reached the desired implant site in the patient (e.g., at or near the annulus of the patient's heart valve that is to be replaced by the prosthetic valve), the prosthetic valve can be deployed or released from the delivery apparatus and re-expanded to full operating size. For balloon-expandable valves, this generally involves releasing the entire valve, assuring its proper location, and then expanding a balloon positioned within the valve stent. For self-expanding valves, on the other hand, the stent automatically expands as the sheath covering the valve is withdrawn. In conventional delivery systems for self-expanding aortic valves, for example, after the delivery system has been positioned for deployment, the annulus end of the valve is typically unsheathed and expanded first, while the aortic end of the valve remains sheathed. Once the annulus end of the valve has expanded, it may be determined that the valve needs to be repositioned in the patient's aortic annulus. To accomplish this, a user (such as a surgeon or an interventional cardiologist) typically re-sheathes the annulus end of the valve, so that the valve can be repositioned while in a collapsed state. After the valve has been repositioned, the user can again release the valve. All these movements in a longitudinal direction are translated into a rotational motion by means of the actuation unit as this is generally known.

In order to ensure a maximum level of sterility with lowest possible sterilization efforts, the handle assembly according to the present invention is disposable and can be replaced by a completely new unit for the next surgery.

The present invention further relates to a corresponding method, according to independent claim 7, of assembling a handle assembly according to the invention for a structural heart catheter based delivery system.

According to the present invention, the step of providing the second bearing element comprises fabricating a tubular body and inserting at least one ball plunger into the tubular body along a longitudinal axis of the body, so that the at least one ball extends from an end face of the tube. This way of assembling the ball bearing element has the advantage that it can easily be accomplished either manually at an end user facility or automatically.

According to a further advantageous embodiment, the second bearing element is fabricated from at least two separate tube segments which are joined along interfaces that extend along said longitudinal axis. Thus, the tube elements have an essentially C-shaped cross-section and can easily be fabricated in a molding process. Furthermore, the attachment of the outer bearing around the inner bearing can be performed quickly and easily. It is clear for a person skilled in the art that the segments may also be connected by means of snap lock devices and/or the use of glue or laser welding. Moreover, at least one of the longitudinal interfaces may be formed by a so-called living hinge (also called film hinge). Living hinges are thin sections of plastic that flexibly connect two segments of a part and are integrally formed with the segments. The materials used to make a living hinge are usually a very flexible plastic such as polypropylene and polyethylene. These can flex more than a million cycles without failure. The advantage of using such a film hinge is that the segments can be attached to each other with less individual parts.

Furthermore, the present invention relates to a method, according to independent claim 9, of testing one or more component(s) of a structural heart catheter based delivery system. In a heart valve delivery system where the occurring forces are not yet known, where new approaches are investigated, and where new untested valve designs are used, the handle assembly according to the present invention can be used to estimate the forces in the delivery system.

Because the handle is serviceable in the field with only two screws, one can quickly add and subtract ball plungers. For instance, the testing routine may start with one pair of ball plungers and the handle is actuated for testing the force. If the handle slips, another ball plunger is added and the testing is repeated until the force needed for actuation is reached. Then the number of ball plungers that are required is counted. A look-up table or chart may be provided to indicate the force value corresponding to the number of ball plungers. This force value will be +/- one ball plunger worth of actual force.

The advantage of this testing method can be seen in the fact that no additional and costly measurement devices are needed and that the method can therefore easily be performed in the field.

The accompanying drawings are incorporated into the specification and form a part of the specification to illustrate several embodiments of the present invention. These drawings, together with the description serve to explain the principles of the invention. The drawings are merely for the purpose of illustrating the preferred and alternative examples of how the invention can be made and used, and are not to be construed as limiting the invention to only the illustrated and described embodiments. Further features and advantages will become apparent from the following more particular description of the various embodiments of the invention, as illustrated in the accompanying drawings, in which like references refer to like elements, and wherein:
- **FIG. 1**: is a schematic perspective representation of a handle assembly according to the present invention;
- **FIG. 2**: is a partly exploded view of the handle assembly of Fig. 1;
- **FIG. 3**: is a schematic representation of a ball plunger used according to the present invention;
- **FIG. 4**: is a schematic sectional view of the ball plunger shown in Fig. 3;
- **FIG. 5**: is a graphic illustration of an exemplary relation between the force threshold value and the amount of balls;
- **FIG. 6**: is a side view of the handle assembly of Fig. 1;
- **FIG. 7**: is a sectional view of the handle assembly of Fig. 1;
- **FIG. 8**: is a perspective sectional view of the handle assembly of Fig. 1;
- **FIG. 9**: is a perspective view illustrating the inner bearing element and the actuation unit.

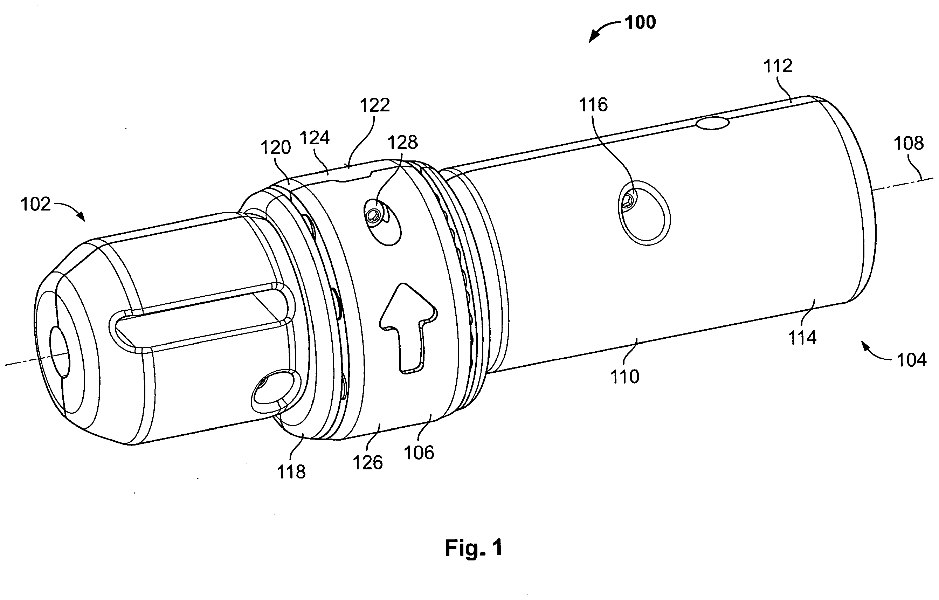

The present invention will now be explained in more detail with reference to the Figures and firstly referring to Fig. 1.

Fig. 1 shows a schematic perspective representation of a handle assembly 100 according to the present invention. The handle assembly has a distal end 102 and a proximal end 104. As used herein, the terms "proximal" and "distal" are to be taken as relative to a user using the disclosed delivery devices. "Proximal" is to be understood as relatively close to the user and "distal" is to be understood as relatively farther away from the user.

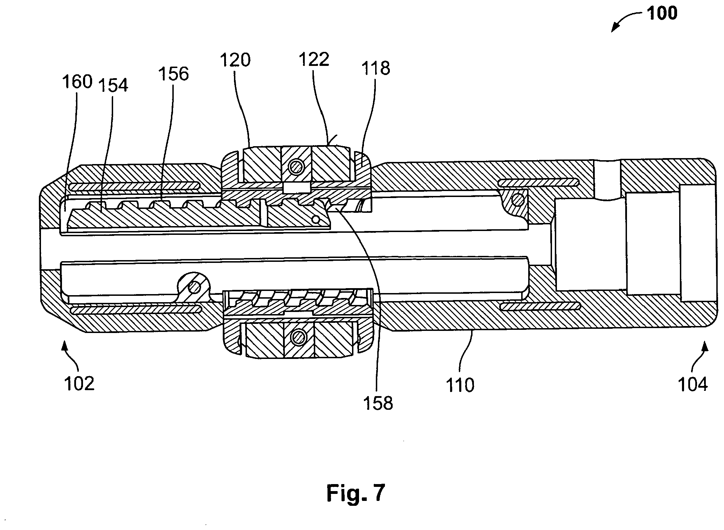

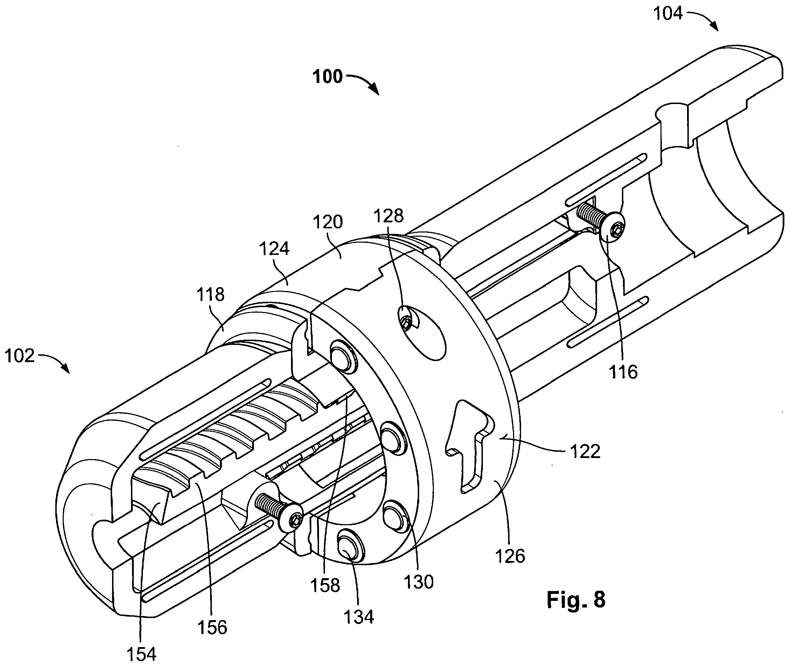

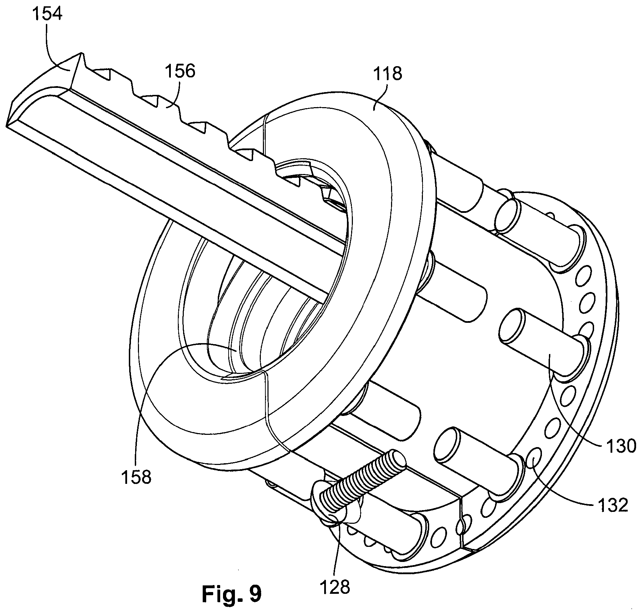

The handle assembly 100 comprises an actuation mechanism (shown in the Figures 7 to 9) that allows a translation of a rotational movement of a wheel element 106 around a longitudinal axis 108 into a linear movement along the longitudinal axis 108. Furthermore, the handle assembly 100 comprises a housing 108 which is formed by a first and a second half-shell 112, 114. The first half-shell 112 and the second half-shell 114 are connected to each other by means of a first screw connection 116. The housing 110 may be fabricated from a plastic material, and may be fabricated by means of injection molding.

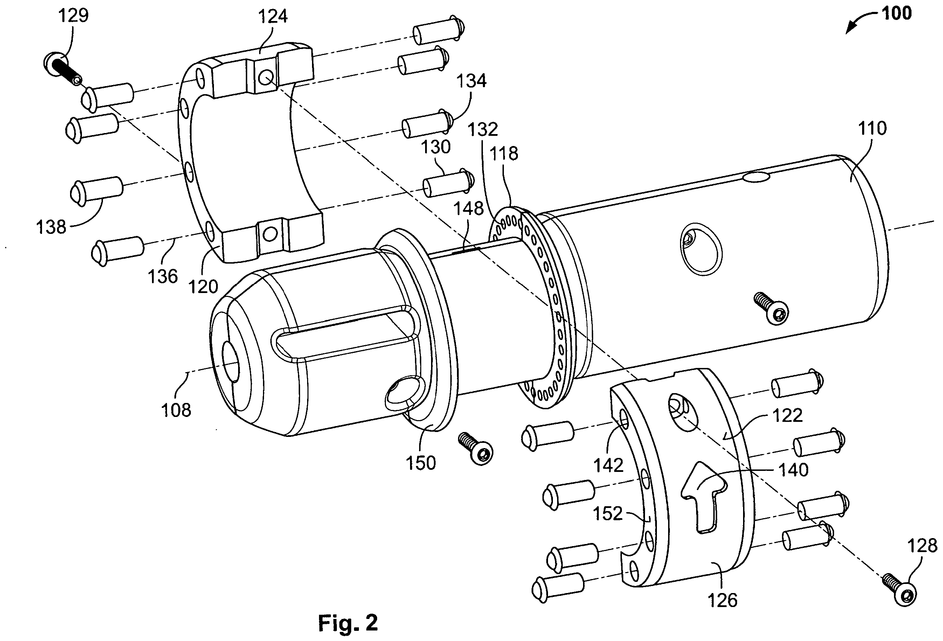

According to the present invention, the wheel element 106 comprises an inner bearing element 118, which is inwardly connected to the actuation unit (not visible in the Figures). The inner bearing element 118 is coupled to an outer bearing element 120 which has an actuation surface 122 that can be touched by a user in order to rotate the wheel element 106. As will be apparent from the following Fig. 2, the outer bearing element 120 is fabricated from two separately fabricated segments 124, 126 which are connected to each other by means of the second screw connection 128.

Fig. 2 shows the handle assembly 100 according to the present invention in a partly exploded representation. As can be seen from this drawing, the inner bearing element 118 is coupled to the outer bearing element 120 via spring-loaded ball plungers 130. To this end, the inner bearing element has a plurality of recesses 132 into which the balls 134 engage. The individual ball plungers 130 are held inside cavities 142 that are provided at the outer bearing element 120.

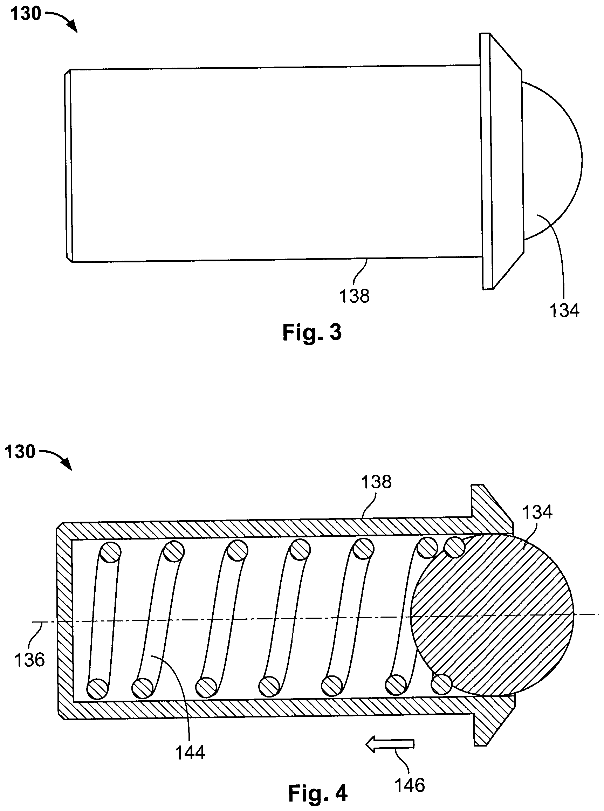

As will be come apparent from Figures 3 and 4, the balls 134 are spring-loaded along their respective longitudinal axes 136, so that they each can be pressed into the bodies 138 of the ball plungers 130. Consequently, when the user turns the wheel element 106 by touching the actuation surface 122 in a rotary direction as indicated by the arrow 140, the outer bearing element 120 and the inner bearing element 118 will stay connected to each other as long as a certain threshold value of the applied torque is not exceeded. Under these conditions, with the inner and the outer bearing elements 118, 120 being coupled, the actuation mechanism inside the handle assembly 100 is actuated by the rotary motion caused by the user.

However, as soon as a particular threshold value of a maximum admissible torque is exceeded, the forces that are exerted on the balls 134 become so high, that each ball 134 is pressed into the body 138 of the ball plunger 130, so that the outer bearing element 120 rotationally slips with respect to the inner bearing element 118. The internal actuation mechanism is no longer actuated by the rotary motion performed by the user, thereby effectively avoiding damaging the actuation mechanism and the components associated therewith. The user advantageously hears and/or senses this slipping between the inner bearing element 118 and the outer bearing element 120 and may reduce the exerted rotational force. By the slipping motion, the balls 134 each exit from the respective recess 132 and glide into a radially adjacent one. Thereby, the inner bearing element 118 and the outer bearing element 120 are coupled with each other again and the internal actuation mechanism may be actuated again by the user touching the actuation surface 122. According to an advantageous embodiment of the present invention, the value of the maximum torque, until which the inner bearing element 118 and the outer bearing element 120 stay mechanically coupled, is determined by the spring constants of the ball plungers 130 and by the total amount of ball plungers 130 used for the coupling. In the embodiment shown in Fig. 2, 16 ball plungers 130 are used. Moreover, as can be seen from Fig. 2, the number of recesses 132 provided at the inner bearing element 118 is higher. Consequently, by using a different outer bearing element 120 with more ball plungers integrated therein, a higher admissible torque may be achieved. However, the number of recesses 132 which are distributed evenly around the circumference of the inner bearing element 118 may of course also exactly correspond to the number of ball plungers 130.

According to a configuration not covered by the present invention, the balls 134 may of course also be assembled directly inside the cavities 142, using springs and balls as separate parts, which are inserted into the cavities 142. However, the fabrication of the handle assembly 100 is significantly facilitated by using prefabricated ball plungers 130.

Furthermore, the outer bearing element 120 is formed by a first segment 124 and a second segment 126. The two segments are attached to each other by means of two screw connections 128, 129. It is clear for a person skilled in the art, however, that more than two segments may also be provided. Furthermore, at least one film hinge may be provided. The advantage of the segmented fabrication is a facilitated injection molding process. Furthermore, as can be seen from Fig. 2, the two half-shells 124, 126 may be formed by two identical parts which are interconnected asymmetrically.

Although not visible in the Figures, each of the cavities 142 is formed by a blind hole. This facilitates mounting the ball plungers 130. However, of course also through holes which extend along the complete length of the outer bearing element 120 along the longitudinal axis 108 can be provided.

Figures 3 and 4 illustrate an example of a ball plunger 130 that is used according to the present invention. Each of the ball plungers 130 has a body 138 which may for instance be made from metal, such as stainless steel. The ball 134 is as assembled inside the body 138 and supported by a spring 144. The diameter of the opening is smaller than the inside diameter of the body 138, so that the ball 134 cannot escape. If a sufficiently high force component is exerted along the axis 136, the ball 134 may be pushed inside the body 138 as indicated by the arrow 146. The force that is necessary for pressing the ball 134 in the direction 146 is determined by the spring characteristics of the spring 144.

Furthermore, the total amount of balls 134 used for coupling the inner coupling 118 with the outer coupling 120 determines the maximum torque that is allowable before the mechanical coupling between the inner bearing 118 and the outer bearing 120 discontinues.

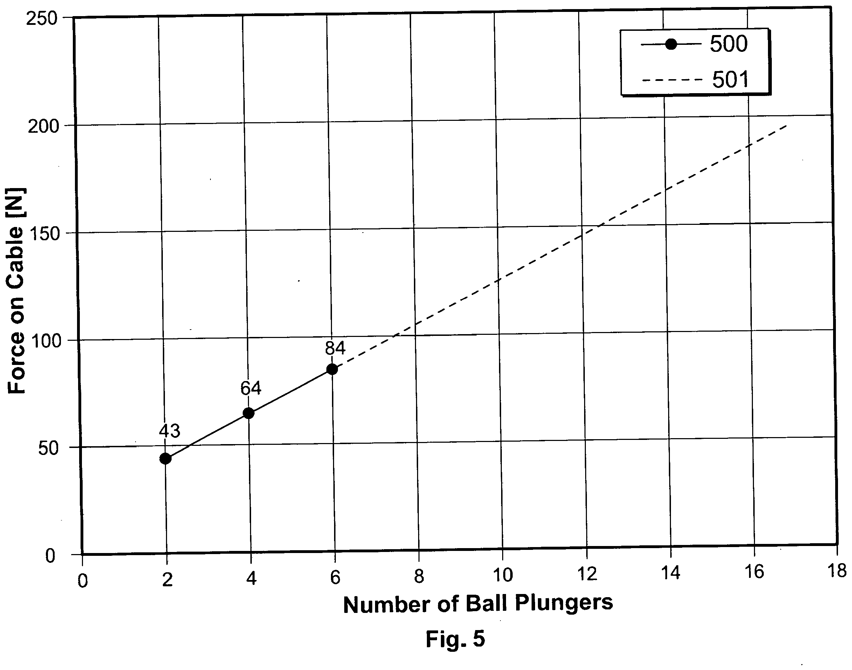

Fig. 5 illustrates the maximum force in N which can be exerted for instance on a deployment cable of a delivery device depending on the amount of ball plungers used. The graph shows a linear correlation between the number of ball plungers and the force inside the handle. Consequently, with just a few measured results the number of plungers needed for a particular handle design can be extrapolated.

In particular, curves 500 indicates the actually performed measurements, while the broken line 501 is a linear extrapolation on the expected force values for higher numbers of ball plungers. As indicated in Fig. 5, using two ball plungers allows a coupling between the inner bearing element 118 and the outer bearing element 120 up to a force of 43 N. Further, using 4 ball plungers keeps the connection established until a threshold value of 64 N is reached. Finally, with a number of six ball plungers, a maximum torque of 84 Newton can be reached before the coupling is disengaged. It is to be expected that this linear dependence will at least be valid also for a number of 16 ball plungers as shown in Fig. 2, so that a threshold value of below 200 N will be reached with this arrangement.

The assembly of a handle according to the present invention will now be explained in detail with reference to figures 1 to 4.

First, the actuation mechanism that translates a rotational motion into a linear motion is mounted inside the housing 110 of the handle assembly 100. Next the inner bearing 118 is mounted at the housing 110 so that it is rotatable with respect of the remainder of the housing 110. The inner bearing 118 may be fabricated from two half-shells which are connected to each other by means of a snap fit connection 148. According to an advantageous embodiment the present invention, the tube shaped inner bearing element 118 is provided with a circumferential collar 150 that has recesses 132 at its inside for engaging with the balls 134.

In a next step, the first half-shells 124 and the second half-shells 126 of the outer bearing element 120, which are provided with cavities 142 at the end faces 152 of the outer bearing element 120, are equipped with a plurality of ball plungers 130. The first half-shell 124 and the second half-shell 126 are attached to the inner bearing element 118 and connected with each other to form a cylindrical tube by mounting the screws 128, 129. In this stage, the balls 134 each engage with one recess 132 while some recesses 132 at the inner bearing element 118 stay empty.

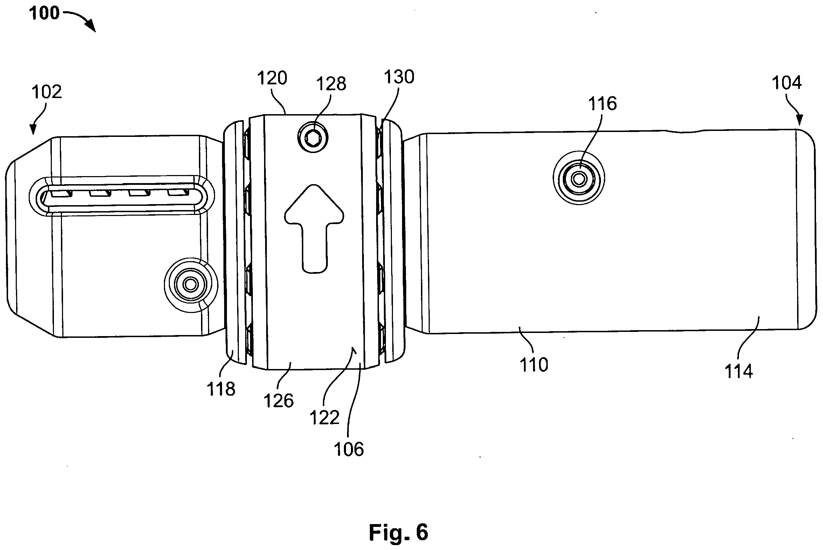

In the following, the actuation mechanism that translates the rotatory movement of the wheel element into a translatory movement will be explained in more detail with reference to Fig. 6 to 9. Fig. 6 illustrates a side view of the handle 100. Fig. 7 shows a longitudinal cut through the handle 100 of Fig. 6. According to an advantageous embodiment of the present invention, the actuation mechanism is based on a screw drive mechanism. As can be seen from Fig. 7, the handle 100 comprises an actuating rod 154 which has a screw thread 156 arranged on its outer surface. The wheel element 106 is formed to be operated as the nut of the screw drive. In particular, the inner bearing 118 of the wheel element 106 has an essentially cylindrical shape and is provided on its inner surface with a nut thread 158. The nut thread 158 is engaged with the screw thread 156, so that a rotating movement of the wheel element 106 (which may also be called "nut") is transformed into an axial movement of the actuating rod 154 along the axis 108.

The actuating rod 154 is arranged in a guiding recess 160 within the housing 110 of the handle 100. As this is known to a person skilled in the art, the actuating rod 154 may be connected to a sheath (not shown in the Figures), which covers a component to be delivered. That is, when the wheel element 106 is in threaded engagement with the threaded actuating rod 154, rotation of the wheel element 106 in one direction (either clockwise or counterclockwise depending on the orientation of the threads on the threaded actuating rod) causes the threaded actuating rod 154 to move proximally within the guiding recess 160, at the same time pulling the sheath in a distal direction to uncover the expandable component.

According to the invention, the user touching the actuation surface 122 can only apply an actuating force until the maximum torque has been reached and the ball plungers 130 disengage from the recesses 132. If the maximum torque has been reached, the actuation surface 122 is decoupled from the actuating rod 154.

In summary, the handle assembly according to the present invention has the advantage that breaking a delivery device by applying a too high torque force can be avoided because the torque limiting bearing prevents the device from reaching the breaking point. Moreover, the design advantageously produces similar results in wet and dry conditions. Furthermore, although in the foregoing description and Figures it has always been assumed that the balls are arranged on the moveable outer part, it is clear for a person skilled in the art that the present invention is not limited to this configuration. According to an alternative of the present invention, the balls can also be arranged on the inner bearing connected to the actuation unit, while the recesses are arranged on the rotatable outer bearing.

Because the handle assembly 100 according to the present invention can easily be disassembled to add or remove one or more ball plunger(s) 130 the handle assembly 100 may further be used for testing new delivery systems. In particular, the present invention relates to a method of testing one or more component(s) of a structural heart catheter based delivery system. In a heart valve delivery system where the occurring forces are not yet known, where new approaches are investigated, and where new untested valve designs are used, the handle assembly 100 according to the present invention as shown in Figures 1 to 4 and 6 to 9 can be used to estimate the forces in the delivery system.

Because the handle 100 is serviceable in the field with only two screws, one can quickly add and subtract ball plungers 130. For instance, the testing routine may start with one pair of ball plungers 130 and the handle 100 is actuated for testing the force. If the handle slips, another ball plunger 130 is added and the testing is repeated until the force needed for actuation is reached. Then the number of ball plungers 130 that are required is counted. A look-up table or chart based on the correlation shown in Fig. 5 may be provided to indicate the force value corresponding to the number of ball plungers 130 which are minimally needed for actuating the actuating unit. This force value will be +/- one ball plunger worth of actual force.

As mentioned above, the testing method according to the present invention comprises the following steps:
(a) providing a handle assembly 100 according to the present invention with a first number of ball plungers 130;
(b) delivering force to the wheel element 106 and determining whether the threshold value of the permissible force has been reached;
(c) if the threshold value has been reached and the wheel element 106 has disengaged from the actuation unit, adding at least one further ball plunger 130, and repeating step (b), or
(d) if the threshold value is no longer reached and the wheel element stays 106 engaged with the actuation unit, counting the number of ball plungers 130 contained in the handle assembly 100, and
(e) determining the force necessary to actuate the delivery system from the smallest number of ball plungers 130 sufficient to keep the wheel element 106 engaged with the actuation unit.

The advantage of this testing method can be seen in the fact that no additional and costly measurement devices are needed and that the method can therefore easily be performed in the field.

**REFERENCE NUMERALS**

| **Reference Numeral** | **Description** |
|---|---|
| 100 | Handle assembly |
| 102 | Distal end of handle assembly |
| 104 | Proximal end of handle assembly |
| 106 | Wheel element |
| 108 | Longitudinal axis |
| 110 | Housing |
| 112 | First half-shell |
| 114 | Second half-shell |
| 116 | First screw connection |
| 118 | Inner bearing element |
| 120 | Outer bearing element |
| 122 | Actuation surface |
| 124 | First segment of outer bearing element |
| 126 | Second segment of outer bearing element |
| 128, 129 | Second screw connection |
| 130 | Ball plunger |
| 132 | Recess |
| 134 | Ball |
| 136 | Axis of ball plunger |
| 138 | Body of ball plunger |
| 140 | Direction of rotary motion |
| 142 | Cavity |
| 144 | Spring |
| 146 | Direction of ball movement |
| 148 | Snap fit connection |
| 150 | Collar |
| 152 | End face |
| 154 | Actuating rod |
| 156 | Screw thread |
| 158 | Nut thread |
| 160 | Guiding recess |
| 500 | Curve indicating measurement values |
| 501 | Curve indicating linear extrapolation |

## Claims

1. Handle assembly for a structural heart catheter based delivery system, the handle assembly (100) comprising:
an actuation unit that is connectable to a delivery member for performing a movement of the delivery member in a longitudinal direction;
an actuating element for manually actuating the actuation unit, wherein the actuating element comprises a wheel element (106) that is rotatable around a rotation axis (108), said rotation axis being the longitudinal axis (108) of the handle assembly (100); wherein
the wheel element (106) is attached to the actuation unit via a force limiting coupling unit (118, 120) for disengaging the wheel element (106) from the actuation unit if a mechanical force applied to the wheel element (106) by a user exceeds a threshold value,
wherein the force limiting coupling unit comprises an inner bearing element (118) that is coupled to the actuation unit and an outer bearing element (120) that is accessible for a user and is engaged with the inner bearing element (118) as long as the mechanical force applied by the user is below said threshold value;
one of the inner bearing element (118) and the outer bearing element (120) has a plurality of bearing recesses (132) each being engaged with a corresponding ball bearing as long as the applied mechanical force does not exceed said threshold value,
the ball bearing comprises a ball plunger (130), the ball plunger (130) having a body (138) with a spring-loaded ball (134) retained therein,
the individual ball plungers (130) are held inside cavities (142) that are provided at the other one of inner bearing element (118) and the outer bearing element (120),
the outer bearing element (120) has an actuation surface (122) that can be touched by a user in order to rotate the wheel element (106).

2. Handle assembly according to claim 1, wherein said outer bearing element (120) comprises at least two segments (124, 126) that are interconnected with each other along an interface that extends along said longitudinal axis (108).

3. Handle assembly according to one of the claims 1 or 2, wherein said outer bearing element (120) has a tube shape with a first and a second end face plane (152) and comprises the plurality of balls (134) which are spring-loaded along said longitudinal axis (108), said balls (134) extending from at least one of the first and second end face planes (152).

4. Handle assembly according to claim 3, wherein said inner bearing element (118) has a tube shape with at least one collar (150) arranged at a peripheral end of the tube around the circumference of the tube, wherein the plurality of bearing recesses (132) which engage with said balls (134) are arranged at the collar (150).

5. Handle assembly according to claim 4, wherein said balls (134) and said bearing recesses (132) are distributed equidistantly around the circumference of the at least one end face plane (152) and the collar (150), respectively, so that each of the balls (134) disengages from one recess (132) when the force exceeds the threshold values and engage with the adjacent bearing recess (132).

6. Delivery system for intravascularly delivering replacement components, the delivery system comprising a handle assembly (100) according to one of the preceding claims.

7. Method of assembling a handle assembly (100) according to one of the claims 1 to 5 for a structural heart catheter based delivery system, the method comprising the following steps:
providing the actuation unit that is connectable to the delivery member for performing the movement of the delivery member in the longitudinal direction;
providing the inner bearing element (118) at the actuation unit, wherein said inner bearing element (118) or the outer bearing element (120) comprises the plurality of bearing recesses (132);
providing the outer bearing element (120) and attaching the outer bearing element (120) at the handle assembly (100), so that it engages with the inner bearing element (118) and forms the wheel element (106) that is rotatable around the rotation axis (108), wherein the outer bearing (120) element or the inner bearing element (118) comprises the plurality of spring loaded balls (134) that each engage with one of the bearing recesses (132) until the mechanical force applied to the wheel element by a user exceeds a threshold value; and
wherein the step of providing one of the inner bearing element (118) and the outer bearing element (120) comprises fabricating a tubular body and inserting the plurality of ball plungers (130) into the tubular body along the longitudinal axis (108) of the body, so that the at least one ball (134) extends from an end face (152) of the tube.

8. Method according to claim 7, wherein said outer bearing element (120) is fabricated from at least two separate tube segments (124, 126) which are joined along interfaces that extend along said longitudinal axis (108).

9. Method for testing a structural heart catheter based delivery system, the method comprising the following steps:
(a) providing a handle assembly according to one of the claims 1 to 5 with a first number of ball plungers;
(b) delivering force to the wheel element and determining whether the threshold value of the permissible force has been reached;
(c) if the threshold value has been reached and the wheel element has disengaged from the actuation unit, adding at least one further ball plunger, and repeating step (b), or
(d) if the threshold value is no longer reached and the wheel element stays engaged with the actuation unit, counting the number of ball plungers contained in the handle assembly, and
(e) determining the force necessary to actuate the delivery system from the smallest number of ball plungers sufficient to keep the wheel element engaged with the actuation unit.

## Patentansprüche

1. Griffanordnung für ein auf einem strukturellen Herzkatheter basierendes Zuführungssystem, wobei die Griffanordnung (100) umfasst:
eine Betätigungseinheit, die mit einem Abgabeelement verbunden werden kann, um eine Bewegung des Abgabeelements in einer Längsrichtung durchzuführen;
ein Betätigungselement zum manuellen Betätigen der Betätigungseinheit, wobei das Betätigungselement ein Radelement (106) umfasst, das um eine Drehachse (108) drehbar ist, wobei die Drehachse die Längsachse (108) der Griffanordnung (100) ist; wobei das Radelement (106) an der Betätigungseinheit über eine Kraftbegrenzungskupplungseinheit (118, 120) zum Lösen des Radelements (106) von der Betätigungseinheit angebracht ist, wenn eine von einem Benutzer auf das Radelement (106) ausgeübte mechanische Kraft einen Schwellenwert überschreitet,
wobei die Kraftbegrenzungskupplungseinheit ein inneres Lagerelement (118), das mit der Betätigungseinheit gekoppelt ist, und ein äußeres Lagerelement (120) umfasst, das für einen Benutzer zugänglich ist und mit dem inneren Lagerelement (118) in Eingriff steht, solange die vom Benutzer ausgeübte mechanische Kraft unterhalb des Schwellenwerts liegt;
eines von dem inneren Lagerelement (118) und dem äußeren Lagerelement (120) weist eine Vielzahl von Lagerausnehmungen (132) auf, die jeweils mit einem entsprechenden Kugellager in Eingriff stehen, solange die ausgeübte mechanische Kraft den Schwellenwert nicht überschreitet,
das Kugellager umfasst einen Kugelstößel (130), wobei der Kugelstößel (130) einen Körper (138) aufweist, in dem eine federbelastete Kugel (134) gehalten wird,
die einzelnen Kugelstößel (130) werden in Hohlräumen (142) gehalten, die an dem jeweils anderen des inneren Lagerelements (118) und des äußeren Lagerelements (120) vorgesehen sind,
das äußere Lagerelement (120) weist eine Betätigungsfläche (122), die von einem Benutzer berührt werden kann, um das Radelement (106) zu drehen.

2. Griffanordnung nach Anspruch 1, wobei das äußere Lagerelement (120) mindestens zwei Segmente (124, 126) umfasst, die entlang einer Schnittstelle, die sich entlang der Längsachse (108) erstreckt, miteinander verbunden sind.

3. Griffanordnung nach einem der Ansprüche 1 oder 2, wobei das äußere Lagerelement (120) eine Rohrform mit einer ersten und einer zweiten Endflächenebene (152) aufweist und die Vielzahl von Kugeln (134) umfasst, die entlang der Längsachse (108) federbelastet sind, wobei sich die Kugeln (134) von mindestens einer der ersten und zweiten Endflächenebene (152) erstrecken.

4. Griffanordnung nach Anspruch 3, wobei das innere Lagerelement (118) eine Rohrform mit mindestens einem Kragen (150) aufweist, der an einem Umfangsende des Rohrs um den Umfang des Rohrs herum angeordnet ist, wobei die mehreren Lagerausnehmungen (132), die mit den Kugeln (134) in Eingriff stehen, an dem Kragen (150) angeordnet sind.

5. Griffanordnung nach Anspruch 4, wobei die Kugeln (134) und die Lagerausnehmungen (132) äquidistant über den Umfang der mindestens einen Endflächenebene (152) bzw. des Kragens (150) verteilt sind, so dass jede der Kugeln (134) aus einer Ausnehmung (132) ausrastet, wenn die Kraft die Schwellenwerte überschreitet, und in die benachbarte Lagerausnehmung (132) eingreift.

6. Zuführungssystem zur intravaskulären Abgabe von Ersatzkomponenten, wobei das Zuführungssystem eine Griffanordnung (100) nach einem der vorhergehenden Ansprüche umfasst.

7. Verfahren zum Zusammenbau einer Griffanordnung (100) nach einem der Ansprüche 1 bis 5 für ein strukturelles Herzkatheter-basiertes Zuführungssystem, wobei das Verfahren die folgenden Schritte umfasst:
Bereitstellung der Betätigungseinheit, die mit dem Abgabeelement verbunden werden kann, um die Bewegung des Abgabeelements in Längsrichtung durchzuführen;
Bereitstellen des inneren Lagerelements (118) an der Betätigungseinheit, wobei das innere Lagerelement (118) oder das äußere Lagerelement (120) die Mehrzahl von Lagerausnehmungen (132) aufweist;
Bereitstellen des äußeren Lagerelements (120) und Befestigen des äußeren Lagerelements (120) an der Griffanordnung (100), so dass es in das innere Lagerelement (118) eingreift und das Radelement (106) bildet, das um die Drehachse (108) drehbar ist, wobei das äußere Lagerelement (120) oder das innere Lagerelement (118) die Mehrzahl von federbelasteten Kugeln (134) umfasst, die jeweils in eine der Lagerausnehmungen (132) eingreifen, bis die von einem Benutzer auf das Radelement ausgeübte mechanische Kraft einen Schwellenwert überschreitet; und
wobei der Schritt des Bereitstellens eines von dem inneren Lagerelement (118) und dem äußeren Lagerelement (120) das Herstellen eines rohrförmigen Körpers und das Einsetzen der Mehrzahl von Kugelstößeln (130) in den rohrförmigen Körper entlang der Längsachse (108) des Körpers umfasst, so dass sich die mindestens eine Kugel (134) von einer Endfläche (152) des Rohrs erstreckt.

8. Verfahren nach Anspruch 7, wobei das äußere Lagerelement (120) aus mindestens zwei separaten Rohrsegmenten (124, 126) hergestellt ist, die entlang von Schnittstellen verbunden sind, die sich entlang der Längsachse (108) erstrecken.

9. Verfahren zum Testen eines auf einem strukturellen Herzkatheter basierenden Zuführungssystems, wobei das Verfahren die folgenden Schritte umfasst:
(a) Versehen einer Griffeinheit nach einem der Ansprüche 1 bis 5 mit einer ersten Anzahl von Kugelstößeln;
(b) Kraft auf das Radelement ausüben und feststellen, ob der Schwellenwert der zulässigen Kraft erreicht wurde;
(c) wenn der Schwellenwert erreicht ist und das Radelement sich von der Betätigungseinheit gelöst hat, Hinzufügen mindestens eines weiteren Kugelstößels und Wiederholen von Schritt (b), oder
(d) wenn der Schwellenwert nicht mehr erreicht wird und das Radelement mit der Betätigungseinheit in Eingriff bleibt, die Anzahl der in der Griffeinheit enthaltenen Kugelstößel zu zählen, und
(e) Bestimmung der Kraft, die erforderlich ist, um das Zuführungssystem mit der kleinsten Anzahl von Kugelstößeln zu betätigen, die ausreicht, das Radelement mit der Betätigungseinheit in Eingriff zu halten.

## Revendications

1. Ensemble manche pour un système de pose basé sur un cathéter cardiaque structurel, l'ensemble manche (100) comprenant :
une unité d'actionnement qui peut être raccordée à un élément de pose pour effectuer un mouvement de l'élément de pose dans un sens longitudinal ;
un élément d'actionnement pour actionner manuellement l'unité d'actionnement, l'élément d'actionnement comprenant un élément roue (106) qui peut tourner autour d'un axe de rotation (108), ledit axe de rotation étant l'axe longitudinal (108) de l'ensemble manche (100) ;
l'élément roue (106) étant fixé à l'unité d'actionnement par l'intermédiaire d'une unité de couplage à limitation de force (118, 120) pour désengager l'élément roue (106) de l'unité d'actionnement si une force mécanique appliquée à l'élément roue (106) par un utilisateur excède une valeur seuil,
l'unité de couplage à limitation de force comprenant un élément de support interne (118) qui est accouplé à l'unité d'actionnement et un élément de support externe (120) qui est accessible pour un utilisateur et qui est engagé avec l'élément de support interne (118) tant que la force mécanique appliquée par l'utilisateur est inférieure à ladite valeur seuil ;
l'un de l'élément de support interne (118) et de l'élément de support externe (120) ayant une pluralité de renfoncements de support (132) chacun étant engagé avec un roulement à billes correspondant tant que la force mécanique appliquée n'excède pas ladite valeur seuil,
le roulement à billes comprenant un poussoir à billes (130), le poussoir à billes (130) ayant un corps (138) ayant une bille à ressort (134) retenue à l'intérieur,
les poussoirs à billes (130) individuels étant maintenus à l'intérieur de cavités (142) qui sont disposées au niveau de l'autre de l'élément de support interne (118) et de l'élément de support externe (120),
l'élément de support externe (120) ayant une surface d'actionnement (122) qui peut être touchée par un utilisateur afin de faire tourner l'élément roue (106).

2. Ensemble manche selon la revendication 1, ledit élément de support externe (120) comprenant au moins deux segments (124, 126) qui sont inter-reliés l'un avec l'autre le long d'une interface qui s'étend le long dudit axe longitudinal (108).

3. Ensemble manche selon l'une des revendications 1 ou 2, ledit élément de support externe (120) ayant une forme de tube ayant un premier et un second plan de face d'extrémité (152) et comprenant la pluralité de billes (134) qui sont à ressort le long dudit axe longitudinal (108), lesdites billes (134) s'étendant depuis au moins l'un du premier et du second plan de face d'extrémité (152).

4. Ensemble manche selon la revendication 3, ledit élément de support interne (118) ayant une forme de tube ayant au moins un collier (150) disposé au niveau d'une extrémité périphérique du tube autour de la circonférence du tube, la pluralité des renfoncements de support (132) qui s'engagent avec lesdites billes (134) étant disposés au niveau du collier (150).

5. Ensemble manche selon la revendication 4, lesdites billes (134) et lesdits renfoncements de support (132) étant distribués de manière équidistante autour de la circonférence du au moins un plan de face d'extrémité (152) et du collier (150), respectivement, de sorte que chacune des billes (134) se désengage d'un renfoncement (132) lorsque la force excède les valeurs seuils et s'engage avec le renfoncement de support (132) adjacent.

6. Système de pose pour composants de remplacement de pose intravasculaire, le système de pose comprenant un ensemble manche (100) selon l'une des revendications précédentes.

7. Procédé d'assemblage d'un ensemble manche (100) selon l'une des revendications 1 à 5 pour un système de pose basé sur un cathéter cardiaque structurel, le procédé comprenant les étapes suivantes :
disposition de l'unité d'actionnement qui peut être raccordée à l'élément de pose pour effectuer le mouvement de l'élément de pose dans le sens longitudinal ;
disposition de l'élément de support interne (118) au niveau de l'unité d'actionnement, ledit élément de support interne (118) ou élément de support externe (120) comprenant la pluralité de renfoncements de support (132) ;
disposition de l'élément de support externe (120) et fixation de l'élément de support externe (120) à l'ensemble manche (100), de sorte qu'il s'engage avec l'élément de support interne (118) et forme l'élément roue (106) qui peut tourner autour de l'axe de rotation (108), l'élément de support externe (120) ou l'élément de support interne (118) comprenant la pluralité de billes à ressort (134) qui s'engagent chacune avec l'un des renfoncements de support (132) jusqu'à ce que la force mécanique appliquée à l'élément roue par un utilisateur excède une valeur seuil ; et
l'étape de disposition de l'un de l'élément de support interne (118) et de l'élément de support externe (120) comprenant la fabrication d'un corps tubulaire et l'insertion de la pluralité des poussoirs à billes (130) dans le corps tubulaire le long de l'axe longitudinal (108) du corps, de sorte que la au moins une bille (134) s'étend depuis une face d'extrémité (152) du tube.

8. Procédé selon la revendication 7, ledit élément de support externe (120) étant fabriqué à partir d'au moins deux segments (124, 126) de tube séparés qui sont joints le long d'interfaces qui s'étendent le long dudit axe longitudinal (108).

9. Procédé de test d'un système de pose basé sur un cathéter cardiaque structurel, le procédé comprenant les étapes suivantes :
(a) disposition d'un ensemble manche selon l'une des revendications 1 à 5 ayant un premier nombre de poussoirs à billes ;
(b) exercice d'une force sur l'élément roue et détermination du fait que la valeur seuil de la force permissible a été atteinte ;
(c) si la valeur seuil a été atteinte et que l'élément roue est désengagé de l'unité d'actionnement, addition d'au moins un poussoir à billes supplémentaire, et répétition de l'étape (b), ou
(d) si la valeur seuil n'est plus atteinte et que l'élément roue reste engagé avec l'unité d'actionnement, décompte du nombre de poussoirs à billes contenus dans l'ensemble manche, et
(e) détermination de la force nécessaire pour actionner le système de pose à partir du plus petit nombre suffisant de poussoirs à billes pour maintenir l'élément roue engagé avec l'unité d'actionnement.
